(19) Europäisches Patentamt
European Patent Office
Office européen des brevets

(11) **EP 3 583 989 B1**

(12) **FASCICULE DE BREVET EUROPEEN**

(45) Date de publication et mention
de la délivrance du brevet:
**10.04.2024 Bulletin 2024/15**

(21) Numéro de dépôt: **19179770.3**

(22) Date de dépôt: **12.06.2019**

(51) Classification Internationale des Brevets (IPC):
**B01D 15/18** *(2006.01)* **C07C 7/12** *(2006.01)*

(52) Classification Coopérative des Brevets (CPC):
(C-Sets disponibles)
**B01D 15/1835; C07C 7/12** (Cont.)

(54) **PROCÉDÉ ET DISPOSITIF DE SÉPARATION EN LIT MOBILE SIMULÉ À DÉBIT DE FLUIDE DE DÉRIVATION**

ABSCHEIDEVERFAHREN UND -VORRICHTUNG IM SIMULIERTEN FLIESSBETT MIT ABLENKUNGSFLÜSSIGKEITSDURCHSATZ

METHOD AND DEVICE FOR SEPARATION IN A SIMULATED MOBILE BED WITH FLOW OF BYPASS FLUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2018 FR 1855449**

(43) Date de publication de la demande:
**25.12.2019 Bulletin 2019/52**

(73) Titulaire: **IFP Energies nouvelles**
**92852 Rueil-Malmaison Cedex (FR)**

(72) Inventeurs:
• **LEINEKUGEL LE COCQ, Damien**
**92852 Rueil-Malmaison Cedex (FR)**
• **HOTIER, Gérard**
**92508 Rueil-Malmaison Cedex (FR)**
• **LE GOFF, Pierre-Yves**
**92508 Rueil-Malmaison Cedex (FR)**
• **LAMBERT, Fabian**
**92508 Rueil-Malmaison Cedex (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
FR-A1- 2 935 100    FR-A1- 2 935 101
FR-A1- 2 944 215    FR-A1- 2 956 037

(52) Classification Coopérative des Brevets (CPC):
(Cont.)

C-Sets
**C07C 7/12, C07C 15/08**

## Description

### Domaine technique

L'invention se rapporte au domaine des séparations de produits naturels ou chimiques, que l'on peut difficilement séparer par distillation. On utilise alors une famille de procédés, et de dispositifs associés, connus sous le nom de procédés, ou dispositifs de séparation en lit mobile simulé, soit en contre-courant simulé, soit en co-courant simulé, que nous désignerons ci-après par l'appellation « LMS ».

Les domaines concernés sont notamment, et de façon non exclusive :

la séparation entre d'une part les paraffines normales et d'autre part les paraffines ramifiées, naphtènes, et aromatiques ;
la séparation oléfines / paraffines ;
la séparation du paraxylène des autres isomères en C8 aromatiques ;
la séparation du métaxylène des autres isomères en C8 aromatiques ; et
la séparation de l'éthylbenzène des autres isomères en C8 aromatiques.

Hors raffinerie et complexe pétrochimique, il existe de nombreuses autres applications parmi lesquelles on peut citer la séparation glucose / fructose, la séparation des isomères de position du crésol, des isomères optiques etc.

### Etat de l'art

La séparation en LMS est bien connue dans l'état de la technique. En règle générale, une colonne fonctionnant en lit mobile simulé comporte au moins trois zones, et éventuellement quatre ou cinq, chacune de ces zones étant constituée par un certain nombre de lits successifs, et chaque zone étant définie par sa position comprise entre un point d'alimentation et un point de soutirage. Typiquement, une colonne en LMS est alimentée par au moins une charge à fractionner et un désorbant (parfois appelé éluant), et l'on soutire de ladite colonne au moins un raffinat et un extrait.

Les points d'alimentation et de soutirage sont modifiés au cours du temps, typiquement décalés dans le même sens d'une valeur correspondant à un lit.

Par définition, on désigne chacune des zones de fonctionnement par un numéro :

zone 1 = zone de désorption des composés de l'extrait, comprise entre l'injection du désorbant et le prélèvement de l'extrait ;
zone 2 = zone de désorption des composés du raffinat, comprise entre le prélèvement de l'extrait et l'injection de la charge à fractionner ;
zone 3 = zone d'adsorption des composés de l'extrait, comprise entre l'injection de la charge et le soutirage du raffinat ; et
optionnellement une zone 4 située entre le soutirage de raffinat et l'injection du désorbant.

L'état de la technique décrit de façon approfondie différents dispositifs et procédés permettant d'effectuer la séparation de charges en lit mobile simulé.

On peut citer notamment les brevets US 2,985,589, US 3,214,247, US 3,268,605, US 3,592,612, US 4,614,204, US 4,378,292, US 5,200,075, US 5,316,821. Ces brevets décrivent également en détail le fonctionnement d'un dispositif LMS.

Les dispositifs LMS comportent typiquement au moins une colonne (et souvent deux), divisée en plusieurs lits d'adsorbant successifs, lesdits lits étant séparés par des plateaux.

Les moyens commandés de distribution et d'extraction de fluides d'un dispositif LMS sont typiquement de l'un des deux grands types suivants de technologie :

soit, pour chaque plateau, une pluralité de vannes commandées tout ou rien pour l'alimentation ou le soutirage des fluides, ces vannes étant typiquement situées au voisinage immédiat du plateau correspondant. Chaque plateau comprend typiquement au moins quatre vannes à deux voies, commandées en tout ou rien, pour effectuer respectivement les alimentations de la charge et du désorbant et les soutirages de l'extrait et du raffinat ;
soit une vanne rotative multi-voies pour l'alimentation ou le soutirage des fluides sur l'ensemble des plateaux.

La présente invention se situe notamment dans le cadre des dispositifs LMS utilisant une pluralité de vannes pour assurer l'alimentation et le soutirage des différents fluides.

Chacun des plateaux comprend typiquement une pluralité de panneaux distributeurs-mélangeurs-extracteurs, dits « plateaux DME » alimentés par des lignes ou systèmes de distribution/extraction. Les plateaux peuvent être de tout type et de toute géométrie. Ils sont généralement divisés en panneaux, correspondant à des secteurs adjacents de la section de la colonne, par exemple des panneaux à secteurs angulaires tels que présentés dans le brevet US 6,537,451 figure 8, ou des panneaux à secteurs parallèles tels que découpés dans une circonférence, ainsi que décrit le brevet US 6,797,175.

La distribution sur chacun des lits requiert une collecte du flux principal provenant du lit précédent, la possibilité d'injecter un fluide annexe ou fluide secondaire tout en mélangeant le mieux possible ces deux fluides, ou encore la possibilité de prélever une partie du fluide collecté, de l'extraire pour l'envoyer vers l'extérieur du dispositif et aussi de redistribuer un fluide sur le lit suivant.

Un problème générique de l'ensemble des dispositifs LMS est de minimiser la pollution générée par le liquide se trouvant dans les différentes zones du ou des circuits d'alimentation et de soutirage de fluides des plateaux, lors des modifications des points d'alimentation et de soutirage au cours du fonctionnement du dispositif LMS.

En effet, lorsque, au cours de la séquence de fonctionnement, une ligne, chambre, ou zone d'alimentation d'un plateau n'est plus balayée par un fluide du procédé, elle devient une zone morte dans lequel le liquide stagne, et n'est remis en mouvement que lorsqu'un autre fluide du procédé y circule à nouveau. De par le fonctionnement du dispositif LMS, il s'agit alors d'un fluide du procédé généralement différent du fluide stagnant dans la ligne considérée.

Le mélange, ou la circulation à bref intervalle de temps de fluides de compositions notablement différentes introduit des perturbations dans le profil de concentration de la zone considérée par rapport au fonctionnement idéal, pour lequel les discontinuités de composition sont à proscrire.

Un autre problème réside dans les éventuelles recirculations entre différentes zones d'un même plateau, et plus généralement de l'ensemble du système de distribution/extraction d'un même plateau, du fait de très petites différences de pression entre les différentes zones du plateau, ce qui induit encore une perturbation par rapport au fonctionnement idéal.

Pour résoudre ces problèmes liés aux recirculations et aux zones mortes, différentes techniques sont connues de l'art antérieur.

Il a déjà été proposé de réaliser un balayage du système de distribution/extraction d'un plateau donné par du désorbant ou du produit recherché, relativement pur. Cette technique permet effectivement d'éviter la pollution du produit désiré lors de son extraction. Toutefois, comme le liquide de balayage a une composition très différente du liquide qu'il déplace, cela introduit des discontinuités de composition préjudiciables au fonctionnement idéal. Cette première variante de balayage réalise typiquement des balayages de courte durée à gradient de concentration élevé. Ces balayages sont de courte durée précisément pour limiter les effets des discontinuités de composition.

Une autre solution consiste, comme décrit dans les brevets US 5,972,224 et US 6,110,364, à faire transiter une majorité du flux principal vers l'intérieur de la colonne et une minorité de ce flux (typiquement de 1 % à 20 % du flux principal) vers l'extérieur par des lignes de dérivation externes entre plateaux successifs. Ce balayage du système de distribution/extraction au niveau d'un plateau par un flux provenant du plateau supérieur est typiquement réalisé en continu, de telle sorte que les lignes et zones du système de distribution/extraction ne soient plus « mortes », mais constamment balayées.

Un tel système avec balayage continu via des lignes de dérivation est présenté à la figure 2 du brevet FR 2,772,634. Les lignes de dérivation sont en général de petit diamètre et comprennent une vanne de petit diamètre, ce qui réduit le coût du système. Un autre procédé est connu de FR 2,956,037.

Selon l'enseignement des brevets US 5,972,224 et US 6,110,364, on cherche à ce que le système de distribution/extraction d'un plateau donné soit balayé par du liquide ayant une composition très voisine de celle du liquide déplacé (liquide présent dans le système de distribution, ou circulant au niveau du plateau). Ainsi, on minimise les mélanges de fluides de composition différente et on réduit les discontinuités de composition.

Dans ce but, les brevets US 5,972,224 et US 6,110,364 préconisent de mettre en oeuvre des débits de balayage dans les dérivations de sorte que la vitesse de transit dans chaque dérivation soit sensiblement la même que la vitesse d'avancement du gradient de concentration dans le flux principal du dispositif LMS. On parle alors de balayage « synchrone », ou « à débit synchrone ». Ainsi, on réalise un balayage des différentes lignes et capacités par un fluide qui a une composition sensiblement identique à celle du liquide qui s'y trouve, et on réintroduit le liquide circulant dans une dérivation en un point où la composition du flux principal est sensiblement identique.

Les balayages sont donc synchrones de longue durée à gradient de concentration faible ou nul. Selon l'enseignement de l'art antérieur, un balayage est dit « synchrone » lorsque le débit de balayage provenant d'un plateau vers le plateau suivant est égal à V/ST dans lequel V est le volume cumulé des systèmes de distribution desdits plateaux, et du volume de la ligne de dérivation entre ces deux plateaux et ST est la période de permutation du dispositif LMS (entre deux permutations successives des alimentations / extractions).

On a donc:

$$\text{« Débit synchrone » } = QS_{i/i+1} = (V_i + V_{i+1} + VL_{i/i+1}) / ST,$$

avec :

QS$_{i/i+1}$ désignant le débit de balayage provenant du plateau P$_i$ vers le plateau voisin (typiquement inférieur) P$_{i+1}$ ;
V$_i$ désignant le volume du système de distribution/extraction du plateau de départ P$_i$ ;
V$_{i+1}$ désignant le volume du système de distribution/extraction du plateau d'arrivée P$_{i+1}$ ;
VL$_{i/i+1}$ désignant le volume de la ligne de dérivation entre P$_i$ et P$_{i+1}$ ; et
ST désignant la période de permutation.

La mise en oeuvre du balayage synchrone est typiquement réalisée à l'aide d'un balayage avec un débit contrôlé, adapté à chacune des zones, allant de 50 % à 150 % du débit synchrone dans ces zones, et idéalement de 100 % du débit synchrone. Les débits dans les lignes de dérivation des 4 zones du dispositif LMS sont contrôlés par des moyens de régulation dans chaque ligne de dérivation.

Le brevet FR 2,935,100 montre qu'il est possible d'améliorer les performances du procédé en réglant les débits des lignes de dérivation dans une zone de fonctionnement donnée en fonction de la présence ou non d'au moins une ligne de dérivation fermée dans ladite zone. Plus précisément, la sur-synchronicité des lignes de dérivation non fermées d'une zone où il existe au moins une ligne de dérivation fermée est définie par le rapport du nombre de lignes de dérivation fermées dans la zone considérée sur le nombre total de lignes de dérivation de la zone, c'est à dire sur le nombre de lits de la zone considérée.

La sur-synchronicité S est définie par la formule suivante:

$$S = a + b\ (nf/nt)$$

dans laquelle :

a est une constante comprise entre -5 et 5,
b est une constante comprise entre 40 et 100,
nf désigne le nombre de lignes de dérivation fermées de la zone considérée, et
nt désigne le nombre total de lignes de dérivation de la zone considérée.

Le brevet FR 2,935,100 nous enseigne par ailleurs qu'il est nécessaire de fermer une ligne de dérivation pour chaque flux injecté (charge et désorbant) et pour chaque flux soutiré (extrait et raffinat).

Les procédés précédemment cités permettent d'obtenir l'objectif de pureté commerciale. Cependant, la demanderesse a pu montrer, que si les enseignements des « balayages synchrones » des brevets US 5,972,224, US 6,110,364 et FR 2,935,100 apportaient une amélioration par rapport à l'art antérieur, il était encore possible, de façon surprenante, d'améliorer encore le fonctionnement et les performances des procédés de séparation en lit mobile simulé.

**Résumé**

Dans le contexte précédemment décrit, un premier objet de la présente description est de fournir un procédé de séparation en lit mobile simulé permettant, à pureté équivalente, d'extraire un soluté de la charge avec un rendement supérieur, en assurant notamment un débit de fuite sur les lignes de dérivation sur lesquelles sont injecté ou soutiré un flux. un deuxième objet est de pourvoir un procédé permettant, à rendement équivalente, d'extraire un soluté de la charge avec une pureté supérieure.

Selon un premier aspect, les objets précités, ainsi que d'autres avantages, sont obtenus par un procédé de séparation en lit mobile simulé d'une charge dans un dispositif de séparation en lit mobile simulé, le dispositif comprenant :

au moins une colonne comprenant une pluralité de lits d'adsorbants séparés par des plateaux comprenant chacun un système de distribution/extraction à deux chambres ; et
des lignes de dérivation externes joignant directement deux plateaux successifs, chaque ligne de dérivation externe comprenant des points d'alimentation de fluides et des points de soutirage d'effluents,
procédé dans lequel :

on alimente l'au moins une colonne avec la charge et un désorbant et on soutire au moins un extrait et au moins un raffinat de l'au moins une colonne, les points d'alimentation et de soutirage étant décalés au cours du temps d'une valeur correspondant à un lit d'adsorbant avec une période de permutation et déterminant une pluralité de zones de fonctionnement du dispositif, et notamment les zones principales suivantes :

une zone 1 de désorption des composés de l'extrait, comprise entre l'alimentation du désorbant et le soutirage de l'extrait,
une zone 2 de désorption des composés du raffinat, comprise entre le soutirage de l'extrait et l'alimentation de la charge,
une zone 3 pour l'adsorption des composés de l'extrait, comprise entre l'alimentation de la charge et le soutirage du raffinat, et
une zone 4 située entre le soutirage du raffinat et l'alimentation du désorbant ;

lorsqu'on injecte un fluide vers un plateau choisi par une ligne de dérivation externe connectée audit plateau choisi, on commande le débit au sein de ladite ligne de dérivation externe de sorte qu'une fraction majoritaire du fluide est injectée vers le plateau choisi, et qu'une fraction minoritaire du fluide est injectée vers le plateau adjacent connecté à ladite ligne de dérivation externe ; et/ou
lorsqu'on soutire un effluent à partir d'un plateau choisi par une ligne de dérivation externe connectée audit plateau choisi, on commande le débit au sein de ladite ligne de dérivation externe de sorte qu'une fraction majoritaire de l'effluent est soutirée à partir du plateau choisi, et qu'une fraction minoritaire de l'effluent est soutirée à partir du plateau adjacent connecté à ladite ligne de dérivation externe, procédé dans lequel
on régule la fraction minoritaire du fluide injectée vers le plateau adjacent connecté à ladite ligne de dérivation externe de sorte qu'un taux de rinçage dudit plateau adjacent est égal à 100 % +/- 30 % ; et/ou on régule la fraction minoritaire de l'effluent soutirée à partir du plateau adjacent connecté à ladite ligne de dérivation externe de sorte qu'un taux de rinçage dudit plateau adjacent est égal à 100 % +/-30 %,

le plateau adjacent étant le plateau adjacent amont disposé en amont du plateau choisi, ou
le plateau adjacent aval disposé en aval du plateau choisi,
le taux de rinçage du plateau adjacent amont étant défini par $Q_{i-1} \times ST / (V_{i-1} + VL_{i-1/i}/2)$,
le taux de rinçage du plateau adjacent aval étant défini par $Q_{i+1} \times ST / (V_{i+1} + VL_{i/i+1}/2)$,
n étant le nombre de lits d'adsorbants de la colonne,
i étant un nombre entier naturel compris entre 1 et n,
$Q_{i-1}$ désignant le débit volumique s'écoulant à partir du plateau adjacent amont,
$Q_{i+1}$ désignant le débit volumique s'écoulant vers le plateau adjacent aval,
$V_{i-1}$ désignant le volume du système de distribution/extraction du plateau adjacent amont,
$V_{i+1}$ désignant le volume du système de distribution/extraction du plateau adjacent aval,
$VL_{i-1/i}$ désignant le volume de la ligne de dérivation externe amont entre le plateau adjacent amont et le plateau choisi,
$VL_{i/i+1}$ désignant le volume de la ligne de dérivation externe aval entre le plateau choisi et le plateau adjacent aval,
ST désignant la période de permutation.

Selon un ou plusieurs modes de réalisation, on régule la fraction minoritaire du fluide injectée vers le plateau adjacent connecté à ladite ligne de dérivation externe de sorte qu'un taux de rinçage dudit plateau adjacent est égal à 100 % +/- 20 % ; et/ou on régule la fraction minoritaire de l'effluent soutirée à partir du plateau adjacent connecté à ladite ligne de dérivation externe de sorte qu'un taux de rinçage dudit plateau adjacent est égal à 100 % +/-20 %.
Selon un ou plusieurs modes de réalisation, on établit dans chaque autre ligne de dérivation externe, une synchronicité à plus ou moins 10 % près.

le débit de synchronicité étant défini par $(V_j + V_{j+1} + VL_{j/j+1}) / ST$,
j étant un nombre entier naturel compris entre 1 et n et différent de i,
n étant le nombre de lits d'adsorbants de la colonne,
i étant un nombre entier naturel compris entre 1 et n ;
$V_j$ et $V_{j+1}$ désignant les volumes respectifs des systèmes de distribution/extraction des plateaux connectés à ladite autre ligne de dérivation externe,
$VL_{j/j+1}$ désignant le volume de ladite autre ligne de dérivation externe,
ST désignant la période de permutation.

Selon un ou plusieurs modes de réalisation, on établit dans chaque autre ligne de dérivation externe, une synchronicité à plus ou moins 5 % près.

Selon un ou plusieurs modes de réalisation, n est un nombre entier naturel compris entre 6 et 24, et de manière préférée entre 8 et 15.

Selon l'invention, chaque plateau est connecté à une ligne de dérivation externe amont entre le plateau adjacent amont et ledit plateau, et une ligne de dérivation externe aval entre ledit plateau et le plateau adjacent aval.

Selon un ou plusieurs modes de réalisation, chaque plateau comprend une pluralité de panneaux distributeurs-mélangeurs-extracteurs de type à secteurs parallèles et alimentations dissymétriques.

Selon un ou plusieurs modes de réalisation, la charge comprend du paraxylène ou du métaxylène au sein d'un mélange d'hydrocarbures aromatiques en C8.

Des modes de réalisation selon le premier aspect ainsi que d'autres caractéristiques et avantages des procédés selon le premier aspect vont apparaître à la lecture de la description qui va suivre, donnée à titre uniquement illustratif et non limitatif, et en référence au dessin suivant.

**Brève description des dessins**

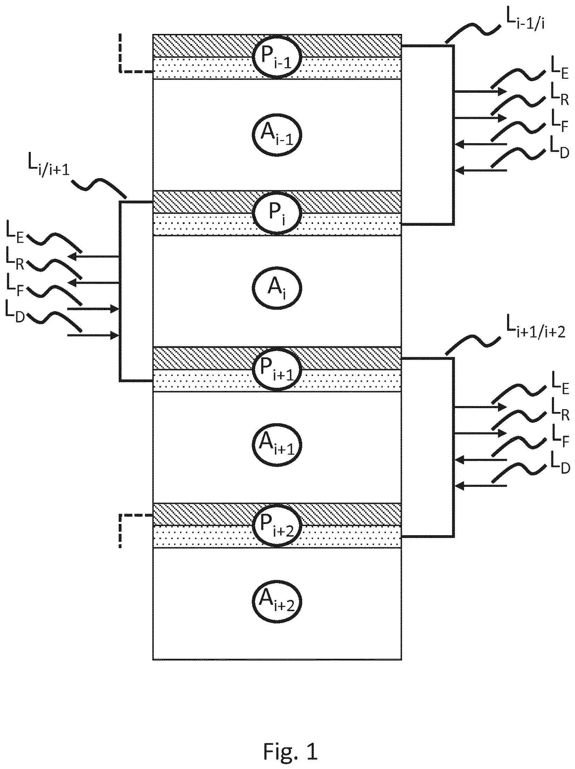

La figure 1 représente un dispositif LMS utilisé dans le procédé selon des modes de réalisation de la présente description, le dispositif comprenant une colonne présentant une succession de plateaux ($P_{i-1}$, $P_i$, $P_{i+1}$, $P_{i+2}$) et de lits ($A_{i-1}$, $A_i$, $A_{i+1}$, $A_{i+2}$), et des lignes de dérivation externes ($L_{i-1/i}$, $L_{i/i+1}$, $L_{i+1/i+2}$).

**Description détaillée**

Le but de l'invention est d'améliorer les performances d'un procédé de séparation en lit simulé par rapport à l'enseignement des brevets US 5,972,224, US 6,110,364 et FR 2,935,100.

En référence à la figure 1, pour améliorer les performances de séparation réalisables par les technologies LMS, l'invention propose un procédé de séparation en LMS d'une charge F dans un dispositif LMS possédant au moins une colonne, ladite colonne étant composée d'une pluralité de lits d'adsorbants $A_i$ séparés par des plateaux $P_i$ comprenant chacun un système de distribution/extraction. Le dispositif LMS comprend en outre des lignes de dérivation externes $L_{i/i+1}$ joignant directement deux plateaux successifs $P_i$, $P_{i+1}$, permettant notamment le balayage desdits plateaux. Chacune de ces lignes de dérivation $L_{i/i+1}$ pouvant comprendre des moyens automatisés de régulation du débit de balayage.

Selon un ou plusieurs modes de réalisation, la colonne comprend n lits d'adsorbants $A_i$. Selon un ou plusieurs modes de réalisation, n est un nombre entier naturel compris entre 6 et 24, préférablement entre 8 et 15, i étant un nombre entier naturel compris entre 1 et n.

Le procédé de séparation en LMS comprend les étapes suivantes : on alimente la charge F et un désorbant D, et on soutire au moins un extrait E, et au moins un raffinat R, les points d'alimentation et de soutirage étant décalés au cours du temps d'une valeur correspondant à un lit d'adsorbant avec une période de permutation (notée ST) et déterminant une pluralité de zones de fonctionnement du dispositif LMS, et notamment les zones principales suivantes :

  une zone 1 de désorption des composés de l'extrait, comprise entre l'alimentation du désorbant D et le soutirage de l'extrait E ;
  une zone 2 de désorption des composés du raffinat, comprise entre le soutirage de l'extrait E et l'alimentation de la charge F ;
  une zone 3 pour l'adsorption des composés de l'extrait, comprise entre l'alimentation de la charge et le soutirage du raffinat R ; et
  une zone 4 située entre le soutirage du raffinat R et l'alimentation du désorbant D,

Il est à noter qu'une ligne de dérivation externe $L_{i/i+1}$ joignant directement deux plateaux successifs $P_i$, $P_{i+1}$, est dite appartenir à une zone lorsque le lit $A_i$ disposé entre les plateaux $P_i$ et $P_{i+1}$ appartient à ladite zone. De plus, les n lits d'adsorbants $A_i$ sont répartis dans les zones 1 à 4 selon des configurations dites de type a / b / c / d, c'est-à-dire que la répartition des lits est la suivante :

  a est le nombre de lits en zone 1 ;
  b est le nombre de lits en zone 2 ;
  c est le nombre de lits en zone 3 ; et
  d est le nombre de lits en zone 4.

Selon un ou plusieurs modes de réalisation :

$$a = (n * 0.208) * (1 \pm 0,2) ;$$

$$b = (n * 0.375) * (1 \pm 0,2) ;$$

$$c = (n * 0.292) * (1 \pm 0,2) ;$$

$$d = (n * 0.125) * (1 \pm 0,2).$$

Le brevet FR 2,935,100 nous enseigne qu'il est nécessaire de fermer une ligne de dérivation pour chaque flux injecté (charge et désorbant) et pour chaque flux soutiré (extrait et raffinat).

Au contraire, dans le procédé selon l'invention, on conserve les lignes de dérivation ouvertes et on contrôle le débit de fuite ainsi généré.

Plus précisément, lorsque l'on injecte un fluide (charge F ou désorbant D) dans un plateau choisi $P_i$, on utilise une ligne d'injection $L_F$ ou $L_D$. Cette ligne est connectée à une ligne de dérivation connectée au dit plateau, c'est à dire soit la ligne de dérivation $L_{i-1/i}$, soit la ligne de dérivation $L_{i/i+1}$. On appelle « plateau adjacent connecté à la dite ligne de dérivation » le plateau $P_{i-1}$ si la ligne de dérivation considérée est la ligne $L_{i-1/i}$, ou le plateau $P_{i+1}$ si la ligne de dérivation considérée est la ligne $L_{i/i+1}$. Quelle que soit la ligne de dérivation connectée à la ligne d'injection utilisée, on contrôle le débit au sein de la dite ligne de manière à ce que la majorité du flux injecté s'écoule vers le plateau choisi $P_i$, et qu'une fraction (non nulle) du flux injecté s'écoule jusqu'au plateau adjacent connecté à la dite ligne de dérivation (e.g. de 1 % à 20 % du flux).

Selon un ou plusieurs modes de réalisation, la fraction minoritaire du flux s'écoulant vers le plateau adjacent connecté à la dite ligne de dérivation est régulée de manière à assurer un taux de rinçage du plateau adjacent connecté à la dite ligne de dérivation égal à 100 % +/-30 %, préférablement égale à 100 % +/- 20 %, de manière préférée égale à 100 % +/- 10 %.

De la même manière lorsque l'on soutire un effluent (extrait E ou raffinat R) dans un plateau choisi $P_i$, on utilise une ligne de soutirage $L_E$ ou $L_R$. Cette ligne de soutirage est connectée à une ligne de dérivation connectée au dit plateau, c'est à dire soit la ligne de dérivation $L_{i-1/i}$, soit la ligne de dérivation $L_{i/i+1}$. On appelle « plateau adjacent connecté à la dite ligne de dérivation » le plateau $P_{i-1}$ si la ligne de dérivation considérée est la ligne $L_{i-1/i}$, ou le plateau $P_{i+1}$ si la ligne de dérivation considérée est la ligne $L_{i/i+1}$. Quelle que soit la ligne de dérivation connectée à la ligne de soutirage utilisée, on contrôle le débit au sein de la dite ligne de manière à ce que la majorité du flux soutiré s'écoule à partir du plateau choisi $P_i$, et qu'une fraction du flux soutiré s'écoule à partir du plateau adjacent connecté à la dite ligne de dérivation.

Selon un ou plusieurs modes de réalisation, la fraction minoritaire du flux s'écoulant à partir du plateau adjacent connecté à la dite ligne de dérivation est régulée de manière à assurer un taux de rinçage du plateau adjacent connecté à la dite ligne de dérivation égal à 100 % +/- 20 %.

Le plateau adjacent au plateau choisi $P_i$ étant soit le plateau adjacent amont ($P_{i-1}$) disposé en amont du plateau choisi ($P_i$), soit le plateau adjacent aval ($P_{i+1}$) disposé en aval du plateau choisi ($P_i$), on définit le taux de rinçage du plateau $P_{i-1}$ par $Q_{i-1} \times ST / (V_{i-1} + VL_{i-1/i}/2)$ et le taux de rinçage du plateau $P_{i+1}$ par $Q_{i+1} \times ST / (V_{i+1} + VL_{i/i+1}/2)$. Dans ces expressions :

$Q_{i-1}$ désigne le débit volumique s'écoulant à partir du plateau $P_{i-1}$ ;
$Q_{i+1}$ désigne le débit volumique s'écoulant vers le plateau $P_{i+1}$ ;
$V_{i-1}$ désigne le volume du système de distribution/extraction du plateau adjacent amont $P_{i-1}$ ;
$V_{i+1}$ désigne le volume du système de distribution/extraction du plateau adjacent aval $P_{i+1}$ ;
$VL_{i/i+1}$ désigne le volume de la ligne de dérivation entre $P_i$ et $P_{i+1}$ ;
$VL_{i-1/i}$ désigne le volume de la ligne de dérivation entre $P_{i-1}$ et $P_i$ ; et
et ST désigne la période de permutation.

On établit dans toutes les autres lignes de dérivation (i.e., les lignes de dérivation dans lesquelles un fluide n'est pas injecté (e.g. charge ou désorbant) ou un effluent n'est pas soutiré (e.g. extrait ou raffinat)) du procédé selon l'invention un débit correspondant à la synchronicité à plus ou moins 10 % près, et de manière préférée à plus ou moins 5 % près, le débit de synchronicité étant défini par $(V_j + V_{j+1} + VL_{j/j+1}) / ST$, expression dans laquelle :

j est un nombre entier naturel compris entre 1 et n et différent de i ;

$V_j$ et $V_{j+1}$ désigne les volumes respectifs des systèmes de distribution/extraction des plateaux ($P_j$ et $P_{j+1}$) connectés à ladite autre ligne de dérivation externe ($L_j/L_{j+1}$) ;

$VL_{j/j+1}$ désigne le volume de la ligne de dérivation entre $P_j$ et $P_{j+1}$ ; et

ST désigne la période de permutation.

Chaque plateau $P_i$ comporte deux chambres permettant d'effectuer les opérations séquentielles d'alimentation de la charge F ou d'injection du désorbant D et d'extraction du raffinat R ou de l'extrait E. La présente invention concerne les colonnes à deux chambres par plateau $P_i$. Plusieurs solutions sont possibles pour l'usage des deux chambres, chacune pouvant être utilisée pour l'injection ou le soutirage d'un ou plusieurs flux. Par exemple, une première chambre peut effectuer les opérations d'injection de charge F ou de désorbant D, et l'autre chambre effectue les opérations de soutirage de raffinat R ou d'extrait E. Un autre cas de figure possible consiste à utiliser une chambre pour l'injection de la charge F et le soutirage du raffinat R, l'autre gérant l'injection de désorbant D et le soutirage de l'extrait E. Ces deux exemples ne sont pas limitants, d'autres utilisations des deux chambres étant possibles. Chaque lit i est équipé d'une ligne de dérivation qui relie une chambre du plateau amont à une chambre du plateau aval.

Selon un ou plusieurs modes de réalisation, la charge est choisie parmi le groupe constitué par un mélange de composés essentiellement aromatiques en C8 (e.g. xylènes et éthylbenzène). Selon un ou plusieurs modes de réalisation, le mélange comprend au moins 95%, préférablement au moins 97% (e.g. au moins 99%) de composés essentiellement aromatiques en C8.

Le procédé selon la présente invention s'applique plus particulièrement à la séparation d'une charge comprenant du paraxylène ou du métaxylène au sein d'un mélange d'hydrocarbures aromatiques en C8. Selon une ou plusieurs modes de réalisation la charge comprend au moins 15% poids de paraxylène et/ou 30% poids [A compléter] de métaxylène par rapport au poids total de la charge.

Un exemple de procédé de séparation LMS de grande importance industrielle concerne la séparation des coupes C8 aromatiques en vue de produire du paraxylène de pureté commerciale, typiquement à au moins 99,7% poids, et un raffinat riche en éthylbenzène, orthoxylène et métaxylène.

Selon un ou plusieurs modes de réalisation, l'adsorbant est choisi parmi le groupe constitué par une zéolithe de type faujasite, de type NaY, BaX, BaKX, BaLSX. Préférablement, l'adsorbant est choisi parmi le groupe constitué par BaX, BaKX, NaY.

Selon un ou plusieurs modes de réalisation, le désorbant est choisi parmi le groupe constitué par un ou plusieurs isomères de diéthylbenzène et le toluène. Préférablement, le désorbant est choisi parmi le groupe constitué par le paradiéthylbenzène et le touène.

Selon un ou plusieurs modes de réalisation, la température de la colonne est comprise entre 120°C et 190°C. Préférablement, la température de la colonne est comprise entre 150°C et 180°C.

Selon un ou plusieurs modes de réalisation, la pression de la colonne est comprise entre 0.3 MPa et 3 MPa. Selon un ou plusieurs modes de réalisation, la pression de la colonne est comprise entre 0.5 MPa et 3 MPa. Selon un ou plusieurs modes de réalisation, la pression de la colonne est comprise entre 0.8 MPa et 3 MPa. Préférablement, la pression de la colonne est comprise entre 1 MPa et 2 MPa.

Selon un ou plusieurs modes de réalisation, la période de permutation ST employée est comprise entre 20 secondes et 120 secondes. Préférablement, la période de permutation ST employée est comprise entre 40 secondes et 100 secondes.

Bien entendu, ces exemples d'application ne sont nullement limitatifs et d'autres applications sont possibles, notamment dans le domaine de la séparation des normales et iso paraffines ou normales et iso oléfines.

### Exemples

L'invention sera mieux comprise à la lecture des exemples qui suivent.

### Exemple 1 (procédé de référence)

On considère un dispositif LMS constitué de 15 lits, de longueur 1,3 m et de rayon interne 3,5 m, avec une ligne d'injection de charge $L_F$, une ligne d'injection de désorbant $L_D$ (pouvant aussi être nommé éluant ou solvant), une ligne de soutirage d'extrait $L_E$ et une ligne de soutirage de raffinat $L_R$. Les plateaux sont à deux chambres de mélange, l'une étant une boîte d'injection (charge F et désorbant D), l'autre étant une boîte de soutirage (extrait E et raffinat R).

Le volume total ($V_i + V_{i+1} + VL_{i/i+1}$), où $VL_{i/i+1}$ est le volume de la ligne de dérivation du plateau $P_i$ au plateau $P_{i+1}$ et où $V_i$ est le volume du système de distribution/extraction du plateau $P_i$, représente 3 % du volume du lit compris entre le plateau $P_i$ et le plateau $P_{i+1}$.

Les lignes de soutirage d'effluents ($L_R$ pour le raffinat R et $L_E$ pour le extrait E) sont situées en aval d'une vanne d'isolement de la ligne de dérivation (on dira plus simplement « en aval de la vanne de ligne de dérivation »). Les lignes d'injection ($L_F$ pour la charge F et $L_D$ pour le désorbant D) sont situées en amont de la vanne d'isolement.

Lorsque l'on injecte un fluide (charge F ou désorbant D) dans le plateau $P_i$, on utilise une ligne d'injection connectée à la ligne de dérivation $L_{i/i+1}$. D'après le procédé de référence, la vanne d'isolement de la ligne de dérivation $L_{i/i+1}$ est fermée pour s'assurer que le fluide injecté s'écoule bien vers le plateau $P_i$.

Lorsque l'on soutire un effluent (extrait E ou raffinat R) dans le plateau $P_i$, on utilise une ligne de soutirage connectée à la ligne de dérivation $L_{i-1/i}$. D'après le procédé de référence, la vanne d'isolement de la ligne de dérivation $L_{i-1/i}$ est fermée pour s'assurer que le fluide injecté s'écoule bien vers le plateau $P_i$.

Il en résulte que l'utilisation de ce type de ligne de dérivation conduit notamment à :

fermer deux lignes de dérivation en zone 1 (injection du désorbant par une ligne connectée à la ligne de dérivation du premier lit de la zone 1, et soutirage de l'extrait par une ligne connectée à ligne de dérivation du dernier lit de la zone 1) ; et

fermer deux lignes de dérivation en zone 3 (injection de la charge par une ligne connectée à la ligne de dérivation du premier lit de la zone 3, et soutirage du raffinat par une ligne connectée à ligne de dérivation du dernier lit de la zone 3).

Les lits sont répartis selon la configuration 3 / 6 / 4 / 2 c'est à dire que la répartition des lits est la suivante :

3 lits en zone 1 ;
6 lits en zone 2 ;
4 lits en zone 3 ; et
2 lits en zone 4.

L'adsorbant employé est une zéolithe de type BaX, et le désorbant est du paradiéthylbenzène. La température de la colonne est de 175°C, et la pression de 1,5 MPa.

La charge est composée de 23 % poids de paraxylène, de 22 % poids d'orthoxylène, de 50 % poids de métaxylène et de 5% poids d'éthylbenzène par rapport au poids total de la charge.

La période de permutation ST employée est de 45 secondes.

Les débits liquides d'injection de charge et de désorbant sont les suivants :

816 $m^3.h^{-1}$ pour la charge ; et
1026 $m^3.h^{-1}$ pour le désorbant,
soit un taux de solvant S/F=1,3.

La synchronicité est réglée à 100 % pour toutes les lignes de dérivation ouvertes.

On obtient par simulation une pureté de paraxylène de 99,76 % et un rendement en paraxylène de 96,27 %.

*Exemple 2 (procédé selon l'invention)*

On considère un dispositif LMS constitué de 15 lits, de longueur 1,3 m et de rayon interne 3,5 m, avec une ligne d'injection de charge $L_F$, une ligne d'injection de désorbant $L_D$ (pouvant aussi être nommé éluant ou solvant), une ligne de soutirage d'extrait $L_E$ et une ligne de soutirage de raffinat $L_R$. Les plateaux sont à deux chambres de mélange, l'une étant une boîte d'injection (charge F et désorbant D), l'autre étant une boîte de soutirage (extrait E et raffinat R).

Le volume total ($V_i + V_{i+1} + VL_{i/i+1}$), où $VL_{i/i+1}$ est le volume de la ligne de dérivation du plateau $P_i$ au plateau $P_{i+1}$ et où $V_i$ est le volume du système de distribution/extraction du plateau $P_i$, représente 3 % du volume du lit compris entre le plateau $P_i$ et le plateau $P_{i+1}$.

Les lignes de soutirage d'effluents ($L_R$ pour le raffinat R et $L_E$ pour le extrait E) sont situées en aval d'une vanne d'isolement de la ligne de dérivation (on dira plus simplement « en aval de la vanne de ligne de dérivation »). Les lignes d'injection ($L_F$ pour la charge F et $L_D$ pour le désorbant D) sont situées en amont de la vanne d'isolement.

Lorsque l'on injecte un fluide (charge F ou désorbant D) dans le plateau $P_i$, on utilise une ligne d'injection connectée à la ligne de dérivation $L_{i/i+1}$. D'après le procédé selon l'invention, le fluide injecté s'écoule majoritairement vers le plateau $P_i$ et une fraction minoritaire du fluide injecté s'écoule au sein de la ligne de dérivation $L_{i/i+1}$ vers le plateau $P_{i+1}$. Cette fraction minoritaire est réglée de manière à assurer un taux de rinçage de 100 % du plateau $P_{i+1}$.

Lorsque l'on soutire un effluent (extrait E ou raffinat R) dans le plateau $P_i$, on utilise une ligne de soutirage connectée à la ligne de dérivation $L_{i-1/i}$. D'après le procédé selon l'invention, le fluide soutiré provient majoritairement du plateau $P_i$ et une fraction minoritaire du fluide soutiré provient du plateau $P_{i-1}$. Cette fraction minoritaire est réglée de manière à assurer un taux de rinçage de 100 % du plateau $P_{i-1}$.

Les lits sont répartis selon la configuration 3 / 6 / 4 / 2 c'est à dire que la répartition des lits est la suivante :

3 lits en zone 1 ;
6 lits en zone 2 ;
4 lits en zone 3 ; et
2 lits en zone 4.

L'adsorbant employé est une zéolithe de type BaX, et le désorbant est du paradiéthylbenzène. La température de la colonne est de 175°C, et la pression de 1,5 MPa.

La charge est composée de 23 % poids de paraxylène, de 22 % poids d'orthoxylène, de 50 % poids de métaxylène et de 5% poids d'éthylbenzène par rapport au poids total de la charge.

La période de permutation ST employée est de 45 secondes.

Les débits liquides d'injection de charge et de désorbant sont les suivants :

816 $m^3.h^{-1}$ pour la charge ; et
1026 $m^3.h^{-1}$ pour le désorbant,
soit un taux de solvant S/F=1,3.

La synchronicité est réglée à 100 % pour toutes les lignes de dérivation ouvertes.

On obtient par simulation une pureté de paraxylène de 99,78 % et un rendement en paraxylène de 97,16 %.

## Revendications

1.  Procédé de séparation en lit mobile simulé d'une charge (F) dans un dispositif de séparation en lit mobile simulé, le dispositif comprenant :

    au moins une colonne comprenant une pluralité de lits d'adsorbants ($A_i$) séparés par des plateaux ($P_i$) comprenant chacun un système de distribution/extraction à deux chambres, chaque plateau ($P_i$) comprenant une première chambre connectée à une ligne de dérivation externe amont ($L_{i-1/i}$) entre le plateau adjacent amont ($P_{i-1}$) et ledit plateau ($P_i$), et une deuxième chambre connectée à une ligne de dérivation externe aval ($L_{i/i+1}$) entre ledit plateau ($P_i$) et le plateau adjacent aval ($P_{i+1}$) ; et
    les lignes de dérivation externes ($L_{i/i+1}$) joignant directement deux plateaux successifs ($P_i$, $P_{i+1}$), chaque ligne de dérivation externe comprenant des points d'alimentation de fluides (F, D) et des points de soutirage d'effluents (E, R),
    procédé dans lequel :

    on alimente l'au moins une colonne avec la charge (F) et un désorbant (D) et on soutire au moins un extrait (E) et au moins un raffinat (R) de l'au moins une colonne, les points d'alimentation et de soutirage étant décalés au cours du temps d'une valeur correspondant à un lit d'adsorbant avec une période de permutation (ST) et déterminant une pluralité de zones de fonctionnement du dispositif, et notamment les zones principales suivantes :

    une zone 1 de désorption des composés de l'extrait, comprise entre l'alimentation du désorbant (D) et le soutirage de l'extrait (E),
    une zone 2 de désorption des composés du raffinat, comprise entre le soutirage de l'extrait (E) et l'alimentation de la charge (F),
    une zone 3 pour l'adsorption des composés de l'extrait, comprise entre l'alimentation de la charge (F) et le soutirage du raffinat (R), et
    une zone 4 située entre le soutirage du raffinat (R) et l'alimentation du désorbant (D) ;

    lorsqu'on injecte un fluide (F, D) vers un plateau choisi ($P_i$) par une ligne de dérivation externe ($L_{i-1/i}$, $L_{i/i+1}$) connectée audit plateau choisi ($P_i$), on commande le débit au sein de ladite ligne de dérivation externe ($L_{i-1/i}$, $L_{i/i+1}$) de sorte qu'une fraction majoritaire du fluide (F, D) est injectée vers le plateau choisi ($P_i$), et qu'une fraction minoritaire du fluide (F, D) est injectée vers le plateau adjacent ($P_{i-1}$, $P_{i+1}$) connecté à ladite ligne de dérivation externe ($L_{i-1/i}$, $L_{i/i+1}$) ; et/ou
    lorsqu'on soutire un effluent (E, R) à partir d'un plateau choisi ($P_i$) par une ligne de dérivation externe ($L_{i-1/i}$, $L_{i/i+1}$) connectée audit plateau choisi ($P_i$), on commande le débit au sein de ladite ligne de dérivation externe ($L_{i-1/i}$, $L_{i/i+1}$) de sorte qu'une fraction majoritaire de l'effluent (E, R) est soutirée à partir du plateau choisi ($P_i$), et qu'une fraction minoritaire de l'effluent (E, R) est soutirée à partir du plateau adjacent ($P_{i-1}$, $P_{i+1}$)

connecté à ladite ligne de dérivation externe ($L_{i-1/i}$, $L_{i/i+1}$),
procédé dans lequel :

on régule la fraction minoritaire du fluide (F, D) injectée vers le plateau adjacent ($P_{i-1}$, $P_{i+1}$) connecté à ladite ligne de dérivation externe ($L_{i-1/i}$, $L_{i/i+1}$) de sorte qu'un taux de rinçage dudit plateau adjacent ($P_{i-1}$, $P_{i+1}$) est égal à 100 % +/- 30 % ; et/ou
on régule la fraction minoritaire de l'effluent (E, R) soutirée à partir du plateau adjacent ($P_{i-1}$, $P_{i+1}$) connecté à ladite ligne de dérivation externe ($L_{i-1/i}$, $L_{i/i+1}$) de sorte qu'un taux de rinçage dudit plateau adjacent ($P_{i-1}$, $P_{i+1}$) est égal à 100 % +/- 30 %,

le plateau adjacent ($P_{i-1}$, $P_{i+1}$) étant le plateau adjacent amont ($P_{i-1}$) disposé en amont du plateau choisi ($P_i$), ou le plateau adjacent aval ($P_{i+1}$) disposé en aval du plateau choisi ($P_i$),
le taux de rinçage du plateau adjacent amont ($P_{i-1}$) étant défini par $Q_{i-1} \times ST / (V_{i-1} + VL_{i-1/i}/2)$,
le taux de rinçage du plateau adjacent aval ($P_{i+1}$) étant défini par $Q_{i+1} \times ST / (V_{i+1} + VL_{i/i+1}/2)$, n étant le nombre de lits d'adsorbants de la colonne,
i étant un nombre entier naturel compris entre 1 et n,
$Q_{i-1}$ désignant le débit volumique s'écoulant à partir du plateau adjacent amont ($P_{i-1}$),
$Q_{i+1}$ désignant le débit volumique s'écoulant vers le plateau adjacent aval ($P_{i+1}$),
$V_{i-1}$ désignant le volume du système de distribution/extraction du plateau adjacent amont ($P_{i-1}$)
$V_{i+1}$ désignant le volume du système de distribution/extraction du plateau adjacent aval ($P_{i+1}$),
$VL_{i-1/i}$ désignant le volume de la ligne de dérivation externe amont ($L_{i-1/i}$) entre le plateau adjacent amont ($P_{i-1}$) et le plateau choisi ($P_i$),
$VL_{i/i+1}$ désignant le volume de la ligne de dérivation externe aval ($L_{i/i+1}$) entre le plateau choisi ($P_i$) et le plateau adjacent aval ($P_{i+1}$),
ST désignant la période de permutation.

2. Procédé selon la revendication 1, dans lequel :

on régule la fraction minoritaire du fluide (F, D) injectée vers le plateau adjacent ($P_{i-1}$, $P_{i+1}$) connecté à ladite ligne de dérivation externe ($L_{i-1/i}$, $L_{i/i+1}$) de sorte qu'un taux de rinçage dudit plateau adjacent ($P_{i-1}$, $P_{i+1}$) est égal à 100 % +/- 20 % ; et/ou
on régule la fraction minoritaire de l'effluent (E, R) soutirée à partir du plateau adjacent ($P_{i-1}$, $P_{i+1}$) connecté à ladite ligne de dérivation externe ($L_{i-1/i}$, $L_{i/i+1}$) de sorte qu'un taux de rinçage dudit plateau adjacent ($P_{i-1}$, $P_{i+1}$) est égal à 100 % +/- 20 %.

3. Procédé selon l'une quelconque des revendications précédentes, dans lequel :
on établit dans chaque autre ligne de dérivation externe ($L_j/L_{j+1}$), une synchronicité à plus ou moins 10 % près,

le débit de synchronicité étant défini par $(V_j + V_{j+1} + VL_{j/j+1}) / ST$,
j étant un nombre entier naturel compris entre 1 et n et différent de i,
n étant le nombre de lits d'adsorbants de la colonne,
i étant un nombre entier naturel compris entre 1 et n,
$V_j$ et $V_{j+1}$ désignant les volumes respectifs des systèmes de distribution/extraction des plateaux ($P_j$ et $P_{j+1}$) connectés à ladite autre ligne de dérivation externe ($L_j/L_{j+1}$),
$VL_{j/j+1}$ désignant le volume de ladite autre ligne de dérivation externe ($L_j/L_{j+1}$),
ST désignant la période de permutation.

4. Procédé selon la revendication 3, dans lequel on établit dans chaque autre ligne de dérivation externe ($L_j/L_{j+1}$), une synchronicité à plus ou moins 5 % près.

5. Procédé selon l'une quelconque des revendications précédentes, dans lequel n est un nombre entier naturel compris entre 6 et 24.

6. Procédé selon l'une quelconque des revendications précédentes, dans lequel chaque plateau ($P_i$) comprend une pluralité de panneaux distributeurs-mélangeurs-extracteurs de type à secteurs parallèles et alimentations dissymétriques.

7. Procédé selon l'une quelconque des revendications précédentes, dans lequel la charge (F) comprend du paraxylène ou du métaxylène au sein d'un mélange d'hydrocarbures aromatiques en C8.

8. Procédé selon la revendication 1, dans lequel la pluralité de lits d'adsorbants (A;) est répartie dans les zones 1 à 4 selon des configurations dites de type a / b / c / d, dans lequel la répartition des lits est la suivante :

a est le nombre de lits en zone 1 ;
b est le nombre de lits en zone 2 ;
c est le nombre de lits en zone 3 ;
d est le nombre de lits en zone 4 ;

$$a = (n * 0.208) * ( 1 \pm 0,2 ) ;$$

$$b = (n * 0.375) * ( 1 \pm 0,2 ) ;$$

$$c = (n * 0.292) * ( 1 \pm 0,2 ) ;$$

et

$$d = (n * 0.125) * ( 1 \pm 0,2 ).$$

9. Procédé selon la revendication 1, dans lequel la colonne comprend n lits d'adsorbants ($A_i$), n étant un nombre entier naturel compris entre 6 et 24.

10. Procédé selon la revendication 1, dans lequel la colonne comprend n lits d'adsorbants ($A_i$), n étant un nombre entier naturel compris entre 8 et 15.

## Patentansprüche

1. Verfahren zur Trennung im simulierten Bewegtbett eines Feeds (F) in einer Vorrichtung zur Trennung im simulierten Bewegtbett,
wobei die Vorrichtung umfasst:

mindestens eine Säule, die eine Mehrzahl von Adsorbensbetten ($A_i$) umfasst, die durch Böden ($P_i$) getrennt sind, die jeweils ein Verteil-/Extraktionssystem mit zwei Kammern umfassen, wobei jeder Boden ($P_i$) eine erste Kammer, die an eine stromaufwärtige externe Bypassleitung ($L_{i-1/i}$) zwischen dem stromaufwärtigen benach-barten Boden ($P_{i-1}$) und dem Boden ($P_i$) angeschlossen ist, und eine zweite Kammer, die an eine stromabwärtige externe Bypassleitung ($L_{i/i+1}$) zwischen dem Boden ($P_i$) und dem stromabwärtigen benachbarten Boden ($P_{i+1}$) angeschlossen ist, umfasst; und
wobei die externen Bypassleitungen ($L_{i/i+1}$) zwei aufeinander folgende Böden ($P_i$, $P_{i+1}$) direkt verbinden, wobei jede externe Bypassleitung Zuführungsstellen für Fluide (F, D) und Abzugsstellen für Abströme (E, R) umfasst, Verfahren, bei dem:
der mindestens einen Säule der Feed (F) und ein Desorbens (D) zugeführt werden und von der mindestens einen Säule mindestens ein Extrakt (E) und mindestens ein Raffinat (R) abgezogen werden, wobei die Zuführungs- und Abzugsstellen im Laufe der Zeit mit einer Umschaltperiode (ST) um einen Wert, der einem Adsorbensbett entspricht, versetzt werden und eine Mehrzahl von Betriebszonen der Vorrichtung bestimmen und insbesondere die folgenden Hauptzonen:

eine Zone 1 zur Desorption der Verbindungen des Extrakts, die zwischen der Zuführung des Desorbens (D) und dem Abzug des Extrakts (E) liegt,
eine Zone 2 zur Desorption der Verbindungen des Raffinats, die zwischen dem Abzug des Extrakts (E) und der Zuführung des Feed (F) liegt,
eine Zone 3 zur Adsorption der Verbindungen des Extrakts, die zwischen der Zuführung des Feeds (F) und dem Abzug des Raffinats (R) liegt, und

eine Zone 4, die zwischen dem Abzug des Raffinats (R) und der Zuführung des Desorbens (D) gelegen ist;

wenn ein Fluid (F, D) zu einem ausgewählten Boden ($P_i$) hin durch eine externe Bypassleitung ($L_{i-1/i}$, $L_{i/i+1}$) zugeführt wird, die an den ausgewählten Boden ($P_i$) angeschlossen ist, der Volumenstrom in der externen Bypassleitung ($L_{i-1/i}$, $L_{i/i+1}$) so gesteuert wird, dass eine mehrheitliche Fraktion des Fluids (F, D) zu dem ausgewählten Boden ($P_i$) hin eingeleitet wird und dass eine minderheitliche Fraktion des Fluids (F, D) zu dem benachbarten Boden ($P_{i-1}$, $P_{i+1}$) hin eingeleitet wird, der an die externe Bypassleitung ($L_{i-1/i}$, $L_{i/i+1}$) angeschlossen ist; und/oder

wenn ein Abstrom (E, R) von einem ausgewählten Boden ($P_i$) aus durch eine externe Bypassleitung ($L_{i-1/i}$, $L_{i/i+1}$) abgezogen wird, die an den ausgewählten Boden ($P_i$) angeschlossen ist, der Volumenstrom in der externen Bypassleitung ($L_{i-1/i}$, $L_{i/i+1}$) so gesteuert wird, dass eine mehrheitliche Fraktion des Abstroms (E, R) von dem ausgewählten Boden (P;) aus abgezogen wird und dass eine minderheitliche Fraktion des Abstroms (E, R) von dem benachbarten Boden ($P_{i-1}$, $P_{i+1}$) aus abgezogen wird, der an die externe Bypassleitung ($L_{i-1/i}$, $L_{i/i+1}$) angeschlossen ist,

Verfahren, bei dem:

die minderheitliche Fraktion des Fluids (F, D), die zu dem benachbarten Boden ($P_{i-1}$, $P_{i+1}$) hin eingeleitet wird, der an die externe Bypassleitung ($L_{i-1/i}$, $L_{i/i+1}$) angeschlossen ist, so reguliert wird, dass eine Spülrate des benachbarten Bodens ($P_{i-1}$, $P_{i+1}$) gleich 100 % +/-30 % ist; und/oder

die minderheitliche Fraktion des Abstroms (E, R), die von dem benachbarten Boden ($P_{i-1}$, $P_{i+1}$) aus abgezogen wird, der an die externe Bypassleitung ($L_{i-1/i}$, $L_{i/i+1}$) angeschlossen ist, so reguliert wird, dass eine Spülrate des benachbarten Bodens ($P_{i-1}$, $P_{i+1}$) gleich 100 % +/- 30 % ist,

wobei der benachbarte Boden ($P_{i-1}$, $P_{i+1}$) der stromaufwärtige benachbarte Boden ($P_{i-1}$) ist, der stromauf des ausgewählten Bodens ($P_i$) angeordnet ist, oder der stromabwärtige benachbarte Boden ($P_{i+1}$), der stromab des ausgewählten Bodens ($P_i$) angeordnet ist,

wobei die Spülrate des stromaufwärtigen benachbarten Bodens ($P_{i-1}$) durch $Q_{i-1} \times ST / (V_{i-1} + VL_{i-1/i}/2)$ definiert wird,

wobei die Spülrate des stromabwärtigen benachbarten Bodens ($P_{i+1}$) durch $Q_{i+1} \times ST / (V_{i+1} + VL_{i/i+1}/2)$ definiert wird,

wobei n die Anzahl von Adsorbensbetten der Säule ist,

wobei i eine natürliche ganze Zahl zwischen 1 und n ist,

wobei $Q_{i-1}$ den Volumenstrom bezeichnet, der von dem stromaufwärtigen benachbarten Boden ($P_{i-1}$) aus fließt,

wobei $Q_{i+1}$ den Volumenstrom bezeichnet, der zu dem stromabwärtigen benachbarten Boden ($P_{i+1}$) hin fließt,

wobei $V_{i-1}$ das Volumen des Verteil-/Extraktionssystems des stromaufwärtigen benachbarten Bodens ($P_{i-1}$) bezeichnet,

wobei $V_{i+1}$ das Volumen des Verteil-/Extraktionssystems des stromabwärtigen benachbarten Bodens ($P_{i+1}$) bezeichnet,

wobei $VL_{i-1/i}$ das Volumen der stromaufwärtigen externen Bypassleitung ($L_{i-1/i}$) zwischen dem stromaufwärtigen benachbarten Boden ($P_{i-1}$) und dem ausgewählten Boden ($P_i$) bezeichnet,

wobei $VL_{i/i+1}$ das Volumen der stromabwärtigen externen Bypassleitung ($L_{i/i+1}$) zwischen dem ausgewählten Boden ($P_i$) und dem stromabwärtigen benachbarten Boden ($P_{i+1}$) bezeichnet,

wobei ST die Umschaltperiode bezeichnet.

2. Verfahren nach Anspruch 1, bei dem:

die minderheitliche Fraktion des Fluids (F, D), die zu dem benachbarten Boden ($P_{i-1}$, $P_{i+1}$) hin eingeleitet wird, der an die externe Bypassleitung ($L_{i-1/i}$, $L_{i/i+1}$) angeschlossen ist, so reguliert wird, dass eine Spülrate des benachbarten Bodens ($P_{i-1}$, $P_{i+1}$) gleich 100 % +/-20 % ist; und/oder

die minderheitliche Fraktion des Abstroms (E, R), die von dem benachbarten Boden ($P_{i-1}$, $P_{i+1}$) aus abgezogen wird, der an die externe Bypassleitung ($L_{i-1/i}$, $L_{i/i+1}$) angeschlossen ist, so reguliert wird, dass eine Spülrate des benachbarten Bodens ($P_{i-1}$, $P_{i+1}$) gleich 100 % +/- 20 % ist.

3. Verfahren nach einem der vorhergehenden Ansprüche, bei dem:

in jeder anderen externen Bypassleitung ($L_j$, $L_{j+1}$) eine Synchronizität auf plus oder minus 10 % genau eingestellt wird,

wobei der Synchronizitätsvolumenstrom durch $(V_j + V_{j+1} + VL_{j/j+1}) / ST$ definiert wird,

wobei j eine natürliche ganze Zahl zwischen 1 und n ist und von i verschieden ist,
wobei n die Anzahl von Adsorbensbetten der Säule ist,
wobei i eine natürliche ganze Zahl zwischen 1 und n ist,
wobei $V_j$ und $V_{j+1}$ die jeweiligen Volumina der Verteil-/Extraktionssysteme der Böden ($P_j$ und $P_{j+1}$) bezeichnen, die an die andere externe Bypassleitung ($L_j$, $L_{j+1}$) angeschlossen sind,
wobei $VL_{j/j+1}$ das Volumen der anderen externen Bypassleitung ($L_j$, $L_{j+1}$) bezeichnet,
wobei ST die Umschaltperiode bezeichnet.

4. Verfahren nach Anspruch 3, bei dem in jeder anderen externen Bypassleitung ($L_j$, $L_{j+1}$) eine Synchronizität auf plus oder minus 5 % genau eingestellt wird,

5. Verfahren nach einem der vorhergehenden Ansprüche, bei dem n eine natürliche ganze Zahl zwischen 6 und 24 ist.

6. Verfahren nach einem der vorhergehenden Ansprüche, bei dem jeder Boden ($P_i$) eine Mehrzahl von Verteil-/Misch-/Extraktionsplatten vom Typ mit parallelen Sektoren und asymmetrischen Zuführungen umfasst.

7. Verfahren nach einem der vorhergehenden Ansprüche, bei dem der Feed (F) Paraxylol oder Metaxylol in einem Gemisch aus aromatischen C8-Kohlenwasserstoffen umfasst.

8. Verfahren nach Anspruch 1, bei dem die Mehrzahl von Adsorbensbetten ($A_i$) in den Zonen 1 bis 4 nach den sogenannten Konfigurationen vom Typ a / b / c / d verteilt ist, wobei die Verteilung der Betten die folgende ist:

a ist die Anzahl von Betten in Zone 1;
b ist die Anzahl von Betten in Zone 2;
c ist die Anzahl von Betten in Zone 3;
d ist die Anzahl von Betten in Zone 4;

$$a = (n * 0{,}208) * (1 \pm 0{,}2);$$

$$b = (n * 0{,}375) * (1 \pm 0{,}2);$$

$$c = (n * 0{,}292) * (1 \pm 0{,}2);$$

und

$$d = (n * 0{,}125) * (1 \pm 0{,}2).$$

9. Verfahren nach Anspruch 1, bei dem die Säule n Adsorbensbetten ($A_i$) umfasst, wobei n eine natürliche ganze Zahl zwischen 6 und 24 ist.

10. Verfahren nach Anspruch 1, bei dem die Säule n Adsorbensbetten ($A_i$) umfasst, wobei n eine natürliche ganze Zahl zwischen 8 und 15 ist.

**Claims**

1. Method for the simulated moving bed separation of a feedstock (F) in a simulated moving bed separation device, the device comprising:

at least one column comprising a plurality of beds of adsorbent ($A_i$) which are separated by plates ($P_i$) each comprising a two-chamber distribution/extraction system, each plate ($P_i$) comprising a first chamber connected to an upstream external bypass line ($L_{i-1/i}$) between the upstream adjacent plate ($P_{i-1}$) and said plate ($P_i$) and a second chamber connected to a downstream external bypass line ($L_{i/i+1}$) between said plate ($P_i$) and the downstream adjacent plate ($P_{i+1}$); and

the external bypass lines ($L_{i/i+1}$) directly joining two successive plates ($P_i$, $P_{i+1}$), each external bypass line comprising fluid (F, D) feed points and effluent (E, R) withdrawal points,
in which method:
the at least one column is fed with the feedstock (F) and a desorbent (D) and at least one extract (E) and at least one raffinate (R) is withdrawn from the at least one column, the feed and withdrawal points being shifted during the course of time by an amount corresponding to one adsorbent bed with a changeover period (ST) and determining a plurality of operating zones of the device, and notably the following main zones:

a zone 1 for the desorption of the compounds from the extract, this zone being comprised between the feed for the desorbent (D) and the withdrawal of the extract (E),
a zone 2 for the desorption of the compounds from the raffinate, this zone being comprised between the withdrawal of the extract (E) and the feed for the feedstock (F),
a zone 3 for the adsorption of the compounds from the extract, this zone being comprised between the feed for the feedstock (F) and the withdrawal of the raffinate (R), and
a zone 4 situated between the withdrawal of the raffinate (R) and the feed for the desorbent (D);
when a fluid (F, D) is injected towards a chosen plate ($P_i$) via an external bypass line ($L_{i-1/i}$, $L_{i/i+1}$) connected to the said chosen plate ($P_i$), the flow rate within the said external bypass line ($L_{i-1/i}$, $L_{i/i+1}$) is controlled in such a way that a major proportion of the fluid (F, D) is injected towards the chosen plate ($P_i$), and that a minor proportion of the fluid (F, D) is injected towards the adjacent plate ($P_{i-1}$, $P_{i+1}$) connected to the said external bypass line ($L_{i-1/i}$, $L_{i/i+1}$); and/or
when an effluent (E, R) is withdrawn from a chosen plate ($P_i$) via an external bypass line ($L_{i-1/i}$, $L_{i/i+1}$) connected to the said chosen plate ($P_i$), the flow rate within the said external bypass line ($L_{i-1/i}$, $L_{i/i+1}$) is controlled in such a way that a major proportion of the effluent (E, R) is withdrawn from the chosen plate ($P_i$), and that a minor proportion of the effluent (E, R) is withdrawn from the adjacent plate ($P_{i-1}$, $P_{i+1}$) connected to the said external bypass line ($L_{i-1/i}$, $L_{i/i+1}$),
in which method:

the minor proportion of the fluid (F, D) injected towards the adjacent plate ($P_{i-1}$, $P_{i+1}$) connected to the said external bypass line ($L_{i-1/i}$, $L_{i/i+1}$) is regulated in such a way that a level of rinsing of the said adjacent plate ($P_{i-1}$, $P_{i+1}$) is equal to 100% +/- 30%; and/or
the minor proportion of the effluent (E, R) withdrawn from the adjacent plate ($P_{i-1}$, $P_{i+1}$) connected to the said external bypass line ($L_{i-1/i}$, $L_{i/i+1}$) is regulated in such a way that a level of rinsing of the said adjacent plate ($P_{i-1}$, $P_{i+1}$) is equal to 100% +/- 30%,
the adjacent plate ($P_{i-1}$, $P_{i+1}$) being the upstream adjacent plate ($P_{i-1}$) positioned upstream of the chosen plate ($P_i$), or the downstream adjacent plate ($P_{i+1}$) positioned downstream of the chosen plate ($P_i$),
the level of rinsing of the upstream adjacent plate ($P_{i-1}$) being defined by $Q_{i-1} \times ST / (V_{i-1} + VL_{i-1/i}/2)$,
the level of rinsing of the downstream adjacent plate ($P_{i+1}$) being defined by $Q_{i+1} \times ST / (V_{i+1} + VL_{i/i+1}/2)$,
n being the number of adsorbent beds in the column,
i being a natural whole number comprised between 1 and n,
$Q_{i-1}$ denoting the volumetric flow rate flowing from the upstream adjacent plate ($P_{i-1}$),
$Q_{i+1}$ denoting the volumetric flow rate flowing towards the downstream adjacent plate (Pi+1),
$V_{i-1}$ denoting the volume of the distribution/extraction system of the upstream adjacent plate ($P_{i-1}$);
$V_{i+1}$ denoting the volume of the distribution/extraction system of the downstream adjacent plate ($P_{i+1}$);
$VL_{i-1/i}$ denoting the volume of the upstream external bypass line ($L_{i-1/i}$) between the upstream adjacent plate ($P_{i-1}$) and the chosen plate ($P_i$),
$VL_{i/i+1}$ denoting the volume of the downstream external bypass line ($L_{i/i+1}$) between the chosen plate ($P_i$) and the downstream adjacent plate ($P_{i+1}$),
ST denoting the changeover period.

2. Method according to Claim 1, wherein:

the minor proportion of the fluid (F, D) injected towards the adjacent plate ($P_{i-1}$, $P_{i+1}$) connected to the said external bypass line ($L_{i-1/i}$, $L_{i/i+1}$) is regulated in such a way that a level of rinsing of the said adjacent plate ($P_{i-1}$, $P_{i+1}$) is equal to 100% +/- 20%; and/or
the minor proportion of the effluent (E, R) withdrawn from the adjacent plate ($P_{i-1}$, $P_{i+1}$) connected to the said external bypass line ($L_{i-1/i}$, $L_{i/i+1}$) is regulated in such a way that a level of rinsing of the said adjacent plate ($P_{i-1}$, $P_{i+1}$) is equal to 100% +/- 20%.

3. Method according to either one of the preceding claims, wherein:
synchronism to within plus or minus 10% is established in each other external bypass line ($L_j/L_{j+1}$),

the synchronism flow rate being defined by ($V_j + V_{j+1} + VL_{j/j+1}$) / ST,
j being a natural whole number comprised between 1 and n and different from i,
n being the number of adsorbent beds in the column,
i being a natural whole number comprised between 1 and n,
$V_j$ and $V_{j+1}$ denoting the respective volumes of the distribution/extraction systems of the plates ($P_j$ and $P_{j+1}$) connected to the said other external bypass line ($L_j/L_{j+1}$),
$VL_{j/j+1}$ denoting the volume of the said other external bypass line ($L_j/L_{j+1}$),
ST denoting the changeover period.

4. Method according to Claim 3, wherein synchronism to within plus or minus 5% is established in each other external bypass line ($L_j/L_{j+1}$).

5. Method according to any one of the preceding claims, wherein n is a natural whole number comprised between 6 and 24.

6. Method according to any one of the preceding claims, wherein each plate ($P_i$) comprises a plurality of distribution-mixing-extraction panels of the parallel sectors type with asymmetric feed.

7. Method according to any one of the preceding claims, wherein the feedstock (F) contains paraxylene or metaxylene within a mixture of C8 aromatic hydrocarbons.

8. Method according to Claim 1, in which the plurality of adsorbent beds ($A_i$) is distributed between zones 1 to 4 in configurations referred to as being of type a / b / c / d, wherein the distribution of the beds is as follows:

a is the number of beds in zone 1;
b is the number of beds in zone 2;
c is the number of beds in zone 3;
d is the number of beds in zone 4;

$$a = (n * 0.208) * (1 \pm 0.2);$$

$$b = (n * 0.375) * (1 \pm 0.2);$$

$$c = (n * 0.292) * (1 \pm 0.2);$$

and

$$d = (n * 0.125) * (1 \pm 0.2).$$

9. Method according to Claim 1, wherein the column comprises n adsorbent beds ($A_i$), n being a natural whole number comprised between 6 and 24.

10. Method according to Claim 1, wherein the column comprises n adsorbent beds ($A_i$), n being a natural whole number comprised between 8 and 15.

Fig. 1

**EP 3 583 989 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2985589 A
- US 3214247 A
- US 3268605 A
- US 3592612 A
- US 4614204 A
- US 4378292 A
- US 5200075 A
- US 5316821 A

- US 6537451 B
- US 6797175 B
- US 5972224 A
- US 6110364 A
- FR 2772634
- FR 2956037
- FR 2935100